# EUROPÄISCHE PATENTSCHRIFT

(11) **EP 1 052 247 B1**
(45) Veröffentlichungstag und Bekanntmachung des Hinweises auf die Patenterteilung: **16.04.2003**
(21) Anmeldenummer: 00109095.0
(22) Anmeldetag: 03.05.2000
(51) Int. Cl.: C07C 239/10

(54) **Verfahren zur Herstellung von (4-Nitrosophenyl)phenylhydroxylamin**
Process for the preparation of (4-Nitrosophenyl)phenylhydroxylamine
Procédé pour la préparation de (4-Nitrosophényl)phénylhydroxylamine

(30) Priorität: 14.05.1999 DE 19922405
(43) Veröffentlichungstag der Anmeldung: 15.11.2000
(73) Patentinhaber: BAYER AG, 51368 Leverkusen (DE)
(72) Erfinder: Giera, Henry, Dr., 86862 Grosskitzighofen (DE); Casser, Carl, Dr., 13585 Berlin (DE); Ooms, Pieter, Dr., 47800 Krefeld (DE); Schelhaas, Michael, Dr., 50733 Köln (DE); Laue, Christian, Dr., 40789 Monheim (DE); Meiers, Michaela, Dr., 50733 Köln (DE); Braun, Gerhard, Dr., 50678 Köln (DE)

(56) Entgegenhaltungen:
- GB-A- 1 257 984

## Beschreibung

Die Erfindung betrifft ein Verfahren zur Herstellung von (4-Nitrosophenyl)-phenylhydroxylamin, das als Zwischenprodukt zur Herstellung von 4-Aminodiphenylamin (4-ADPA), einem wichtigen Einsatzprodukt zur Synthese von Alterungsschutzmitteln und Stabilisatoren in der Gummi- und Polymerindustrie (Kirk-Othmer, Encyclopedia of Chemical Technology, 4^{th} Edition, 1992, Vol 3,.Seite 424-456; Ullmann's Encylopedia of Industrial Chemistry, 5^{th} Edition, Vol A3, 1985, Seite 91-111) verwendet werden kann.

Synthesen für (4-Nitrosophenyl)phenylhydroxylamin sind bisher nur ausgehend von Nitrosobenzol beschrieben. Nitrosobenzol wird im stark sauren Medium zu (4-Nitrosophenyl)phenylhydroxylamin dimerisiert, wenn Säuren wie Schwefelsäure (Bamberger et al., Chem. Ber., 31 (1898), 1513; DE 2020043), Peroxytrifluoressigsäure (Boyer, J.Org.Chem. 24 (1959) 2038), Flußsäure (Wiechert, Z.Chem. 15 (1955) 21; DE 1147237), Sulfonsäuren, Perchlorsäure, Trifluoressigsäure (DE 2703919) und Lewis-Säuren, wie z.B. BF₃xOEt₂ (EP 9267) eingesetzt werden.

Auch bei der Umsetzung von Nitrosobenzol mit Alkoholaten als Basen wird (4-Nitrosophenyl)phenylhydroxylamin als Nebenprodukt gebildet (Aurich et al., Liebigs Ann. Chem. (1981) 1271-1281; Hutton, Waters, J.Chem.Soc.(B) (1968) 191).

Ein gravierender Nachteil der beschriebenen Verfahren besteht darin, daß sie von Nitrosobenzol ausgehen, das großtechnisch nicht wirtschaftlich hergestellt werden kann, insbesondere nicht aus dem wohlfeilen Nitrobenzol (Kochetova, Klyuyev, Petroleum Chemistry, Vol.37, (1997) 414-421).

Es war daher wünschenswert ein Verfahren zur Herstellung von (4-Nitrosophenyl)phenylhydroxylamin zu entwicklen, das von einem billigeren und wohlfeileren Ausgangsmaterial als Nitrosobenzol ausgeht.

Gegenstand der vorliegenden Erfindung ist ein Verfahren zur Herstellung von (4-Nitrosophenyl)phenylhydroxylamin, das dadurch gekennzeichnet ist, daß Nitrobenzol mit hydroxid- und/oder oxidgruppenhaltigen Basen gegebenenfalls in Anwesenheit eines Lösungsmittels, bei Temperaturen von 20 bis 180°C und Drücken von 0,1 bis 10 bar umgesetzt wird.

Als für das erfindungsgemäße Verfahren geeignete hydroxid-und/oder oxidgruppenhaltige Basen kommen in Frage anorganische Basen,wie Alkalimetallhydroxide, Alkalimetalloxide, Erdalkalimetallhydroxide, Erdalkalimetalloxid sowie die entsprechenden Hydroxide und Oxide der Elemente 58 bis 71 des Periodensystems der Elemente (nach IUPAC, neu).Beispielsweise werden genannt: Die Oxide und Hydroxide von Natrium, Kalium, Lithium, Caesium, Magnesium, Kalzium, Barium, Lanthan und/oder Cer, insbesondere die Oxide und Hydroxide von Lithium, Natrium, Kalium, Caesium, ganz besonders bevorzugt Caesiumhydroxid.

Weiterhin kommen organische Basen in Frage,wie beispielsweise quartäre Alkylammonium-hydroxide (NR₄⁺OH⁻mit R unabhängig voneinander für Alkyl, Aryl oder Aralkyl mit 1 bis 7 Kohlenstoffatomen). Als Beispiele seien genannt: Tetramethylammoniumhydroxid, Tetraethylammoniumhydroxid, Tetrapropylammoniumhydroxid, Tetrabutylammonium-hydroxid, Methyltributylammoniumhydroxid, Methyltripropylammoniumhydroxid, Methyltriethylammoniumhydroxid, Trimethylbenzylammoniumhydroxid. Besonders bevorzugt sind Tetrapropylammoniumhydroxid und Tetrabutylammoniumhydroxid. Ganz besonders bevorzugt wird Tetramethylammoniumhydroxid eingesetzt.

Selbstverständlich ist es auch möglich, die Basen in Mischungen untereinander einzusetzen. Das jeweils günstigste Mischungsverhältnis kann dabei leicht durch entsprechende Vorversuche ermittelt werden. Weiterhin ist es möglich, die anorganischen Basen in Verbindung mit Phasentransferkatalysatoren einzusetzen. Geeignete Phasentransferkatalysatoren sind beispielsweise beschrieben in W.E. Keller, Fluka-Kompendium, Bd.1,2,3, Georg Thieme Verlag, Stuttgart 1986, 1987, 1992. Beispielsweise können die zuvor erwähnten Basen mit Kronenether, wie 18-Krone-6 oder quartären Ammoniumverbindungen, zusammen eingesetzt werden. Die erfindungsgemäß einzusetzenden Basen können einen Wassergehalt von bis zu 6 mol Wasser, bevorzugt bis zu 3 mol Wasser, besonders bevorzugt bis zu 2,5 mol Wasser, bezogen auf ein Mol Base, besitzen. Ein höherer Wassergehalt verschlechtert im allgemeinen die Ausbeuten.

Die erfindungsgemäßen Basen können in fester Form, als Schmelze oder als Lösung oder Gemisch in einem Lösungsmittel oder Lösungsmittelgemisch zugesetzt werden.

Erfindungsgemäß werden die Basen in Mengen von 0,01 bis 3, bevorzugt 0,1 bis 2 Äquivalente pro Mol Nitrobenzol eingesetzt.

Als Lösungsmittel kommen aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatomen sowie Amide mit 1 bis 10 Kohlenstoffatomen in Frage. Selbstverständlich können die erwähnten Lösungsmittel im Gemisch untereinander eingesetzt werden. Als geeignete Lösungsmittel werden insbesondere genannt: Benzol, Toluol, Xylol, tert.-Butylmethylether, tert.-Amylmethylether, Diisopropylether, Diethylenglykoldimethylether, Glycoldimethylether, Dioxan, Tetrahydrofuran, Diamylether, Chlorbenzol, Dichlorbenzol, Dimethylformamid, Dimethylacetamid und N-Methylpyrolidinon. Bevorzugt werden eingesetzt, Toluol, Xylol, Glykoldimethylether, tert.-Butylmethylether, Diisopropylether, Diethylenglykoldimethylether, insbesondere tert.-Butylmethylether und Toluol. Die Menge an Lösungsmittel ist für das erfindungsgemäße Verfahren nicht kritisch. Die geeignetste Menge kann ebenfalls leicht durch entsprechende Vorversuche ermittelt werden. Die Lösungsmittelmenge hängt insbesondere ab von der Reaktionstemperatur und von der Art und Menge der eingesetzten Basen und Katalysatoren. Üblicherweise werden die Lösungsmittel in Mengen von 1 bis 99 Gew.-%, bevorzugt 5 bis 95 Gew.-%, besonders bevorzugt 15 bis 90 Gew.-%, bezogen auf die Gesamtmenge der Reaktionsmischung, eingesetzt.

Die Reaktionstemperaturen bei dem erfindungsgemäßen Verfahren betragen bevorzugt 20 bis 180°C, insbesondere 50 bis 150°C.

Es ist von Vorteil den Gehalt an protischem Material, wie z.B. Wasser zu kontrollieren, was durch wasserbindende Reagenzien, wie Molekularsiebe, KOH, P₂O₅, aber auch durch Strippen mit inertem Gas, durch Abdestillieren von Wasser bei z.B. vermindertem Druck und bevorzugt durch azeotrope Destillation erreicht wird.

Es ist ebenfalls von Vorteil den Sauerstoffgehalt in der Reaktionslösung zu minimieren, was durch Entgasen der Lösungen und durch übliche Schutzgastechnik mit z.B. Stickstoff und/oder Argon erreicht werden kann.

Die Isolierung des gewünschten (4-Nitrosophenyl)phenylhydroxylamins aus dem Reaktionsgemisch erfolgt gegebenenfalls nach vorheriger Filtration oder Neutralisation durch Kristallisation. Es ist jedoch auch möglich das erhaltene Reaktionsgemisch ohne weitere Aufarbeitung für weitere Umsetzungen zu verwenden.

Insbesondere ist das erhaltene Reaktionsgemisch, in Anwesenheit von Wasserstoff und Hydrierkatalysatoren geeignet , 4-ADPA herzustellen, welches wiederum zur Herstellung von Alterungsschutzmitteln, insbesondere in der Kautschukindustrie dient.

### Beispiele

### Beispiel 1

123 g Nitrobenzol werden in 300 g Di-n-butylether bei 25°C vorgelegt. Zu dieser Lösung werden 140 g Tetramethylammoniumhydroxid-dihydrat zugegeben. Unter Inertgas-atmosphäre wird das Reaktionsgemisch vier Stunden auf 75-80°C erwärmt. Mittels GC (interner Standard) und HPLC (externer Standard) wird bei einem Umsatz von 78% eine Selektivität von 16 % an (4-Nitrosophenyl)phenylhydroxylamin bestimmt. Nach Zugabe von 50 ml Wasser werden die Phasen getrennt. Die wäßrige Phase wird durch Zugabe von 25 %iger Schefelsäure auf pH = 1 gestellt und mehrfach mit Methylenchlorid extrahiert. Nach Einengung im Vakuum verbleibt ein Rückstand von 85 g mit einem (4-Nitrosophenyl)phenylhydroxylamingehalt von 14 % (HPLC; externer Standard). Dies entspricht einer Selektivität von 16 %.

### Beispiel 2

7.4 g Tetramethylammoniumhydroxid-dihydrat und 10 g Molekularsieb wurden in 50 ml Di-n-butylether vorgelegt Unter Inertgasatmosphäre wurde auf 80°C erwärmt, 6.2 g Nitrobenzol zugegeben und 5 Stunden bei dieser Temperatur geführt. Nach Zugabe von 50 ml Wasser konnten bei 98% Umsatz in der polaren Phase 17% (4-Nitrosophenyl)phenylhydroxylamin mit HLPC bestimmt werden.

### Beispiel 3

7.4 g Tetramethylammoniumhydroxid-dihydrat wurden in 50 ml Triethylamin vorgelegt. Unter Inertgasatmosphäre wurde auf 80°C erwärmt, 6.2 g Nitrobenzol zugegeben und 5 Stunden bei dieser Temperatur gerührt. Nach Zugabe von 50 ml Wasser konnten bei 84 % Umsatz in der polaren Phase 14% (4-Nitrosophenyl)phenylhydroxylamin mit HLPC bestimmt werden.

### Beispiel 4

22.2 g Tetramethylammoniumhydroxid-dihydrat wurden in 50 ml tert-Amyl-methylether vorgelegt. Unter Inertgasatmosphäre wurde auf 80°C erwärmt, 6.2 g Nitrobenzol zugegeben und 2 Stunden bei dieser Temperatur gerührt. Nach Zugabe von 50 ml Wasser konnten bei 100 % Umsatz in der polaren Phase 9 % (4-Nitrosophenyl)phenylhydroxylamin mit HLPC bestimmt werden.

### Beispiel 5

7.4 g Tetramethylammoniumhydroxid-dihydrat und 10 g Molekularsieb wurden in 50 ml Tetrahydrofuran vorgelegt. Unter Inertgasatmosphäre wurde zum Rückfluß erhitzt, 6.2 g Nitrobenzol zugegeben und 5 Stunden unter Rückfluß gekocht. Nach Zugabe von 50 ml Wasser konnten bei 77% Umsatz in der polaren Phase 8% (4-Nitrosophenyl)phenylhydroxylamin mit HLPC bestimmt werden.

### Beispiel 6

3.7 g Tetramethylammoniumhydroxid-dihydrat und 10 g Molekularsieb wurden in 50 ml Di-n-butylether vorgelegt. Unter Inertgasatmosphäre wurde auf 80°C erwärmt, 6.2 g Nitrobenzol zugegeben und 3 Stunden bei dieser Temperatur gerührt. Nach Zugabe von 50 ml Wasser konnten bei 75% Umsatz in der polaren Phase 5% (4-Nitrosophenyl)phenylhydroxylamin mit HLPC bestimmt werden.

### Beispiel 7

7.4 g Tetramethylammoniumhydroxid-dihydrat und 10 g Molekularsieb wurden in 50 ml tert-Butylmethylether vorgelegt. Unter Inertgasatmosphäre wurde zum Rückfluß erhitzt, 6.2 g Nitrobenzol zugegeben und 3 Stunden unter Rückfluß gekocht. Nach Zugabe von 50 ml Wasser konnten bei 65% Umsatz in der polaren Phase 7% (4-Nitrosophenyl)phenylhydroxylamin mit HLPC bestimmt werden.

### Beispiel 8

7.4 g Tetramethylammoniumhydroxid-dihydrat und 10 g Molekularsieb wurden in 50 ml Toluol vorgelegt. Unter Inertgasatmosphäre wurde auf 80°C erwärmt, 6.2 g Nitrobenzol zugegeben und 5 Stunden bei dieser Temperatur gerührt. Nach Zugabe von 50 ml Wasser konnten bei 95% Umsatz in der polaren Phase 8% (4-Nitrosophenyl)phenylhydroxylamin mit HLPC bestimmt werden.

### Beispiel 9

7.4 g Tetramethylammoniumhydroxid-dihydrat und 10 g Molekularsieb wurden in 50 ml Di-n-butylether vorgelegt. Unter Inertgasatmosphäre wurde auf 65°C erwärmt, 6.2 g Nitrobenzol zugegeben und 2 Stunden bei dieser Temperatur gerührt. Nach Zugabe von 50 ml Wasser konnten bei 88% Umsatz in der polaren Phase 12% (4-Nitrosophenyl)phenylhydroxylamin mit HLPC bestimmt werden.

### Beispiel 10

7.2 g Tetramethylammoniumhydroxid-dihydrat wurden in 50 ml tert-Amyl-methylether vorgelegt. Unter Inertgasatmosphäre wurde auf 80°C erwärmt, 6.2 g Nitrobenzol zugegeben und 3 Stunden bei dieser Temperatur gerührt. Nach Zugabe von 50ml Wasser konnten bei 76% Umsatz in der polaren Phase 12% (4-Nitrosophenyl)phenylhydroxylamin mit HLPC bestimmt werden.

### Beispiel 11

7.5 g Tetramethylammoniumhydroxid-dihydrat wurden in einem Gemisch 50 ml Di-n-butylether und 10 ml Triethylamin vorgelegt. Unter Inertgasatmosphäre wurde auf 80°C (Badtemperatur) erwärmt, 6.2 g Nitrobenzol zugegeben und 5 Stunden bei dieser Temperatur gerührt. Nach Zugabe von 25 ml Wasser konnten bei 79% Umsatz in der polaren Phase 15% (4-Nitrosophenyl)phenylhydroxylamin mit HLPC bestimmt werden.

### Beispiel 12

61.6 g Tetramethylammoniumhydroxid-dihydrat wurden in 48 ml tert-Amyl-methylether vorgelegt. Unter Inertgasatmosphäre wurde auf 80°C erwärmt, 63.6 g Nitrobenzol zugegeben und 4.5 Stunden bei dieser Temperatur gerührt. Aus der polaren Phase konnten bei 67% Umsatz in der polaren Phase 6% (4-Nitrosophenyl)phenylhydroxylamin mit HLPC bestimmt werden.

### Beispiel 13

85 g des Rückstandes aus Beispiel 1 werden in 200 g Isopropanol gelöst und nach Zugabe von 5 g Pd/C (5 %ig) bei 50°C und 30 bar H2 bis zur Beendigung der Wasserstoffaufnahme hydriert. Nach Filtration des Katalysators werden 0,055 mol 4-ADPA im Filtrat nachgewiesen (GC, interner Standard). Dies entspricht einer Selektivität von 92 % bezogen auf (4-Nitrosophenyl)phenylhydroxylamin.

## Patentansprüche

1. Verfahren zur Herstellung von (4-Nitrosophenyl)phenylhydroxylamin, **dadurch gekennzeichnet, daß** man Nitrobenzol in Gegenwart von hydroxidund/oder oxidhaltigen Basen bei Temperaturen von 20 bis 180°C und Drücken von 0,1 bis 10 bar umsetzt.

2. Verfahren nach Anspruch 1, **dadurch gekennzeichnet, daß** man die Umsetzung bei Temperaturen von 50 bis 150°C durchführt.

3. Verfahren nach Anspruch 1 und 2, **dadurch gekennzeichnet, daß** man als hydroxid- und/oder oxidgruppenhaltige Basen Alkalihydroxide, Alkalimetalloxide, Erdalkalimetallhydroxide, Erdalkalimetalloxide, die Hydroxide und Oxide der Elemente 58 bis 71 des Periodensystems der Elemente (IUPAC) sowie quartäre Alkylammonium-hydroxide und -alkoholate einsetzt.

4. Verfahren nach einem der Ansprüche 1 bis 3 **dadurch gekennzeichnet, daß** die hydroxid und /oder oxidgruppenhaltige Base einen Phasentransferkatalysator kombiniert mit einer Basenquelle darstellt

5. Verfahren nach einem der Ansprüche 1 bis 4 **dadurch gekennzeichnet, daß** man die Basen in Mengen von 0,01 bis 3 Äquivalente pro Mol Nitrobenzol einsetzt.

6. Verfahren nach einem der Ansprüche 1 bis 5, **dadurch gekennzeichnet, daß** der Wassergehalt im Reaktionsgemisch durch Entfernen des Wassers kontrolliert wird.

7. Verfahren nach Anspruch 1, **dadurch gekennzeichnet, dass** man die Umsetzung in Anwesenheit von Lösungsmitteln durchführt.

## Claims

1. A process for preparing (4-nitrosophenyl)phenylhydroxylamine, **characterised in that** nitrobenzene is reacted in the presence of hydroxide and/or oxide-containing bases at temperatures of 20 to 180°C and pressures of 0.1 to 10 bar.

2. A process according to Claim 1, **characterised in that** the reaction is performed at temperatures of 50 to 150°C.

3. A process according to Claim 1 and 2, **characterised in that** alkali metal hydroxides, alkali metal oxides, alkaline earth metal hydroxides, alkaline earth metal oxides, the hydroxides and oxides of elements 58 to 71 of the Periodic System of Elements (IUPAC) and quaternary alkylammonium hydroxides and alcoholates are used as hydroxide and/or oxide group-containing bases.

4. A process according to one of Claims 1 to 3, **characterised in that** the hydroxide and/or oxide group-containing base is a phase transfer catalyst combined with a source of a base.

5. A process according to Claims 1 to 4, **characterised in that** the bases are used in amounts of 0.01 to 3 equivalents per mol of nitrobenzene.

6. A process according to one of Claims 1 to 5, **characterised in that** the water content of the reaction mixture is controlled by removing water.

7. A process according to Claim 1, **characterised in that** the reaction is performed in the presence of solvents.

## Revendications

1. Procédé de préparation de la (4-nitroso-phényl)phénylhydroxylamine, **caractérisé en ce que** le nitrobenzène est mis à réagir en présence de bases contenant des radicaux hydroxyle et/ou oxyde, à des températures allant de 20 à 180°C et sous des pressions allant de 0,1 à 10 bar.

2. Procédé suivant la revendication 1, **caractérisé en ce que** l'on réalise la réaction à des températures allant de 50 à 150°C.

3. Procédé suivant les revendications 1 et 2, **caractérisé en ce que** l'on met en oeuvre comme base contenant des radicaux hydroxyle et/ou oxyde, des hydroxydes de métal alcalin, des oxydes de métal alcalin, des hydroxydes de métal alcalino-terreux, des oxydes de métal alcalino-terreux, les hydroxydes et oxydes des éléments 58 à 71 du système périodique des éléments (IUPAC) ainsi que les hydroxydes et alcoolates d'alcoylammonium quaternaire.

4. Procédé suivant l'une quelconque des revendications 1 à 3, **caractérisé en ce que** la base contenant des radicaux hydroxyde et/ou oxyde représente un catalyseur de transfert de phase combiné à une source basique.

5. Procédé suivant l'une quelconque des revendications 1 à 4, **caractérisé en ce que** l'on met en oeuvre les bases en des quantités allant de 0,01 à 3 équivalents par mole de nitrobenzène.

6. Procédé suivant l'une quelconque des revendications 1 à 5, **caractérisé en ce que** la teneur en eau du mélange réactionnel est contrôlée par élimination de l'eau.

7. Procédé suivant la revendication 1, **caractérisé en ce que** l'on réalise la réaction en présence de solvants.
